# EUROPÄISCHE PATENTSCHRIFT

(11) **EP 0 657 178 B1**
(45) Veröffentlichungstag und Bekanntmachung des Hinweises auf die Patenterteilung: **03.07.2002**
(21) Anmeldenummer: 94117912.9
(22) Anmeldetag: 14.11.1994
(51) Int. Cl.: A61L 27/00, C12N 5/00

(54) **Verfahren zur Herstellung von Implantatkeramikmaterial, insbesonders von Hydroxylapatit aufweisenden Implantatkeramikmaterial**
Method for making implant ceramic material especially hydroxyapatite containing implant ceramic material
Procédé de préparation de matériau céramique pour implant en particulier matériau céramique pour implant contenant de l'hydroxyapatite

(30) Priorität: 30.11.1993 SE 9303977
(43) Veröffentlichungstag der Anmeldung: 14.06.1995
(73) Patentinhaber: CORIMED GmbH, D-64404 Bickenbach (DE)
(72) Erfinder: Otten, Klaus, D-64404 Bickenbach (DE)
(74) Vertreter: Wagner, Karl Heinz

(56) Entgegenhaltungen:
- EP-A- 0 303 941
- EP-A- 0 382 047
- EP-A- 0 475 189
- DATABASE WPI Week 9235 Derwent Publications Ltd., London, GB; AN 92-288770 XP002125026 & JP 04 198007 A (KANEBO), 17. Juli 1992 (1992-07-17)

## Beschreibung

Die Anwendung von Hydroxylapatit aufweisenden Implantatmaterial in der medizinischen Versorgung von Patienten bei Defekten im oder am Knochengerüst hat sich nach anfänglichen vorsichtigen Versuchen als effektiv erwiesen. Die in der Anfangszeit der Implantate aus Calciumphosphat-Keramik erlittenen medizinischen Rückschläge sind nunmehr identifiziert worden. Ursächlich waren vor allem die synthetischen Herstellungsmethoden, ganz speziell die Verwendung dichter Keramiken, sowie die Unkenntnis der realen kristallinen Zusammensetzung und die zum grössten Teil ungeeigneten Sintertemperaturen.

Diesen nachteiligen Wirkungen der Calciumphosphat-Keramiken wurde mit der Entwicklung der biologischen Knochenersatzwerkstoffe auf Hydroxylapatitbasis erfolgreich entgegengewirkt. Die Einführung von Knochenersatzkeramiken hergestellt aus biologischen Ausgangsmaterialien wie Algen, Korallen und Knochen haben hier einen grundlegenden Wandel in der Akzeptanz dieser Keramiken bewirkt. Speziell die Entwicklung von den sogenannten Knochenkeramiken mit ihrer hervorragenden Übereinstimmung in Struktur und Zusammensetzung haben den Sektor der Knochenersatzwerkstoffe auf Basis von Hydroxylapatitkeramik sowohl medizinisch als auch wirtschaftlich in den Vordergrund des Interesses gerückt.

So hat eine Folge von Entwicklungen auf dem Gebiet der Hydroxylapatitkeramiken bovinen Ursprunges eine Reihe von technologischen Entwicklungen ausgelöst die in Patentschriften und Marktprodukten heute als Stand der Technik zu bezeichnen sind.

Grundsätzlich wird von bovinen Materialien ausgegangen, die über ein jeweils unterschiedliches Verfahren erst enteiweist werden und daran anschliessend thermisch gesintert werden. Der Ursprung dieser Entwicklungen liegt sicherlich in dem in der Patentschrift DE-C-961 654 beschriebenen Material, der als Kieler Knochenspan bekannt wurde, jedoch aus arzneimittelrechtlichen Gründen nicht mehr eingesetzt werden darf. Nach dem in der Patentschrift EP 0 141 004 beschriebenen Methode wird der Knochen erst von seinen Weichteilen befreit, daran anschliessend auf chemischen Wege enteiweist (10%ige H₂O₂-Lösung) und daran anschliessend gesintert wird. Ähnliches wird in der Patentschrift US-A-4 314 380 beschrieben, wobei hier neben H₂O₂ auch NaOH zum Einsatz gelangt und die Sintertemperaturen nicht erreicht werden. Das in der Patentschrift DE 37 27 606 beschriebene Verfahren geht von einer schonenden pyrolytischen Entfernung der Organik aus, wobei nach einer schonenden Trocknung eine Pyrolyse unter Luftunterschuss oder unter reduzierender Atmosphäre mit anschliessender oxidativen Sinterung erfolgt. Nach dem in der DE 40 28 683 offengelegten Verfahren werden Stabilitätsprobleme aus dem Verfahren nach DE 37 27 606 durch Behandlung mit organischen Säuren zwischen Pyrolyse und Sinterung behoben.

Allen Verfahren gemeinsam ist die schonende Entfernung der Organik, entweder mittels chemischer Hilftsmittel oder reduzierender Atmosphäre und zusätzlich die Behandlung mit Säure in einem Zwischenschritt in Bezug auf das zuletzt genannte Verfahren. Gemeinsam ist auch die anschliessende Sinterung.

Die wesentlichen Nachteile aller dieser Verfahren ist entweder die Verwendung chemischer Zusätze zur Herstellung, beziehungsweise die energetisch und zeitlich aufwendige schonende Entfernung der Organik in der Pyrolyse.

Aufgabe der Erfindung war nun in einem ersten Schritt jedwede Verwendung chemischer Substanzen bei der Herstellung zu vermeiden, den energieaufwendigen Teil der schonenden Pyrolyse wesentlich zu verbessern, auch unter den Aspekten des Umweltschutzes und zusätzlich die Variation der Kristallgrössen und die Einstellung der Basizität und Porosität zu ermöglichen.

Erfindungsgemäss wird die Aufgabe dahingehend gelöst, dass zur Herstellung von der erfindungsgemäss neuen Knochenkeramik die spongiösen Bereiche der Gelenkkugeln von Tieren, insbesonders ausgewachsenen Rindern einem sehr schnellen Temperaturprozess unter mehrfachem Luftüberschuss unterzogen wird, ohne dass dabei die Struktur der spongiösen Bälkchen geschädigt wird. Daran anschliessend erfolgt eine Lagerung, beziehungsweise eine permanente oder interruptierende Behandlung mit Wasser zur natürlichen Reduzierung von überschüssigem Calcium, und/ /oder nach der anschliessenden klassischen Sinterung eine weitere Lagerung oder Benebelung mit Wasser.

In Bezug auf die Porosität hat sich die Verwendung von Tieren, inbesonders von ausgewachsenen Rindern zu Gewinnung der spongiösen Bereiche der Bein und Armgelenke als besonders vorteilhaft erwiesen, da die Trabekelstruktur voll ausgebildet ist und weite Bereiche mit gleichmässiger Porenstruktur vorliegen. Die durch die keramische Sinterung hervorgerufene Schwindung in den drei Dimensionen bewirkt, dass bei der Verwendung der oben beschriebenen Gelenkteile die vom Rind herstammende überdimensionale Porengrössenverteilung auf die Spongiosaporendimensionensverteilung humaner Spongiosa reduziert wird.

Überraschenderweise hat es sich gezeigt, dass ein nicht vorbehandelter Knochen einer raschen Temperatureinwirkung ohne Strukturschädigung unterzogen werden kann und der Krematoriumseffekt des Zerfallens des Knochens unterbunden werden kann. Erfindungsgemäss wird die Verkürzung der Entfernung der Organik dadurch erreicht, dass ein kaltes, bzw. gefrorenes Knochenstück schlagartig in den heissen Brennofen geschoben wird und dort für eine kurze Zeit bei erhöhtem Luftwechsel belassen wird. Hierbei brennt die Organik von aussen nach innen ab, so dass die Aussenseite schon organikfrei ist, wenn die Innenseite noch gefroren ist. Dadurch kann effektvoll verhindert werden, dass schmelzendes und verkokendes Fett oder Gewebe die Poren abschliesst und sich im Inneren des Teiles ein zerstörerischer Überdruck aufbauen kann. Dieser Vorgang erfolgt in wenigen Minuten und bewirkt gleichzeitig die Deckung des grössten Teiles des Energiebedarfes für diesen Verfahrensschritt, da die Verbrennung der Organik stark exotherm abläuft, womit die erfindungsgemässe Zeitersparnis bei der Entfernung der Organischen Bestandteile erfüllt ist und ausserdem die Aufwendungen an Energie erfindungsgemäss drastisch reduziert wurden. Im Sinne des aktiven Umweltschutzes sind die Verwendung von Luft im Überschuss in Verbindung mit den entstehenden Verbrennungsgasen geeignet eine katalytische Abgasnachverbrennung zu unterhalten, die ihrerseits wieder mit ihrer Abwärme einen Beitrag zur Vorwärmung der Verbrennungsluft leistet. Ebenfalls erfindungsgemässer Effekt bei dieser schnellen Verbrennung ist, dass die Kristalle der anorganischen Matrix des eingesetzten Knochens sich in ihrer Ausgangsgrösse nur unwesentlich verändern, da den Kristallen keine/kaum Zeit zu Kristallwachstum gegeben wird. Dadurch wird erreicht, dass die Erzeugung der später gewünschten Kristallitgrösse annähernd von der Ausgangskristallitgrösse ausgehen kann, so dass die minimale Kristallitgrösse im späteren Endprodukt nur knapp über der Ausgangskristallitgrösse liegen kann. Dieser Herstellungsschritt bewirkt die Entfernung der organischen Matrix mit Verfestigung der anorganischen Bestandteile, wobei hier noch nicht von einem Sintereffekt, sondern lediglich von einem in der Keramik als Schrühen (Verfestigung einer Matrix ohne das Eintreten von Sintermerkmalen) bezeichneter Vorgang stattfindet. Mit der optional anschliessenden Lagerung oder Besprühung mit reinem Wasser werden die nicht an dem Knochengerüst anhaftenden anorganischen Bestandteile herausgewaschen und gleichzeitig leicht lösliche Komponenten reduziert und homogenisiert. Daran anschliessend erfolgt die klassische keramische Sinterung, wobei die verwendeten Zeiten und Temperaturen auf die Zielkristallitgrösse abgestimmt werden können, ebenso wie die Bindungsfestigkeit und der Grad der Sinterung durch entsprechende Zeit- und Temperaturwahl eingestellt werden können. Optional zu dem ersten Lager, bzw. Benebelungsbad kann jetzt ein weiteres Bad in Wasser oder die Besprühung mit Wasser erfolgen. Mit dieser Abschlussspühlung wird das Mass der oberflächlich verfügbaren Bestandteile, die die Basizität in einem Medium bewirken sukzessive reduziert. Der Effekt dieser Wasserbehandlung nach dem Sintervorgang ist die Möglichkeit der Einstellung der Basizität der Implantatkeramik von z.B. pH 6,0 bis hin zu pH 13, der nach erneuter Temperaturbehandlung stabilisiert werden kann. Die Formgebung und Verpackung des daraus resultierenden Implantatmaterials kann auf alle dem Stand der Technik entsprechende Methoden erfolgen.

Im Nachfolgenden wird exemplarisch für die Vielzahl der nach diesem Verfahren mögliche Parameter die Herstellung eines Hydroxylapatitkeramikimplantates beschrieben:

Aus dem Hüftgelenk eines ausgewachsenen Rindes wird die epiphysäre Spongiosa durch Zersägen des Gelenkes in 2,7 cm * 2,7 cm beliebig lange Streifen gewonnen. Die Epiphysenstreifen werden über einen Zeitraum von 10 Stunden bei -18 Grad Celsius eingefroren. Die tiefgefrorenen Epiphysenstreifen werden nun auf einen feuerfesten Schlitten gelegt und unverzüglich in einen mit heisser Luft vorgeheizten Brennofen-Kammertemperatur beim Einschieben 650 Grad Celsius, in dem permanent ein Luftsauerstoffüberschuss von mehr als 2 herscht, schnell eingeschoben. Der Vorgang des Einschiebens dauert 2 Sekunden. Nun wird der in der Umwandlung befindliche Epiphysenknochen exakt 10 Minuten in dieser Atmosphäre belassen, wobei die neu zugeführte Heissluft den Epiphysenknochen direkt an- bzw. durchströmt. Die durch direkte Verbrennung entstehende Abwärme und Abgase werden der katalytischen Abgasnachverbrennung zugeführt, die ihrerseits mit ihrer Abwärme die neu zugeführte Luft heizt. Nach Ablauf der 10 Minuten wird der Epiphysenknochen aus dem Brennofen herausgezogen und abgekühlt. Der nun von seinem Fett och Gewebe befreite anorganiche Teil des Epiphysenknochens, in dem maximal Restbestände an Kohlenstoff vorliegen, hat eine derart ausreichende Festigkeit, dass er ohne Probleme über 1,5 Stunden mit Wasser besprüht werden kann. Das Ablaufwasser kann dabei wieder aufbereitet werden und dem Prozess neu zugeführt werden. Der vorgebrannte und gewaschene Epiphysenknochen wird nun unter Luftatmosphäre mit 2 Kelvin pro Minute auf eine Endtemperatur von 1250 Grad Celsius erhitzt, die dann für eine Dauer von 90 Minuten aufrechterhalten wird. Nach dem Abkühlen wird die frisch gesinterte Epiphysenknochenkeramik nochmals über einen Zeitraum von 6 Stunden mit Wasser besprüht. Das Ablaufwasser kann ebenfalls nach einer Aufbereitung wiederverwendet werden. Daran anschliessend erfolgt eine Formgebung durch abschneiden der Epiphysenkeramikstreifenendstücke, so dass ein Teil mit einem Mass von 2 cm * 2 cm * 5 cm resultiert. Das entstandene Implantat besitzt eine Qualität, wie sie von der ASTM für Hydroxylapatitkeramik als Implantationsmaterial gefordert wird. Direkt vor der Verpackung wird das Epiphysenkeramikimplantat nochmals kurzfristig auf 900 Grad erhitzt und in sterilen Glasbehältern in einer sterilen Atmosphäre eingepackt. Ist keine Sterilabfüllung möglich, werden die Teile daran anschliessend einer Strahlensterilisation, einer Gassterilisation oder einer thermischen Sterilisation unterworfen.

Die Variationsbreite der Herstellung von Implantatkeramiken nach dieser Methode fängt bereits bei der Wahl der Knochen an. Bewährt für die Herstellung haben sich die Epiphysenbereiche und die Metaphysenbereiche der Gelenkinnenteile von Hüftgelenk, Kniegelenk, Schultergelenk und Ellbogengelenk von ausgewachsenen Rindern, wobei die Epiphysenspongiosa des Hüftgelenkes die beste Keramik liefert. In der Vorbereitung der Knochen für die Hochgeschwindigkeitsverbrennung liegen der Variationsbereiche von erhöhter Raumtemperatur bis zum bei -80 Grad Celsius gefrorenen Temperaturbereich. Beste Ergebnisse wurden mit einer Temperatur von ca. -15 Grad Celsius erreicht. In der Hochgeschwindigkeitsbrennkammer kann die Temperatur knapp über der Zündtemperatur des Knochens liegen und darf maximal bis zur Phasenumwandlungstemperatur von Hydroxylapatit erhöht werden. Versuche mit Temperaturen zwischen 650 Grad Celsius und 900 Grad Celsius zeigten hier gute energetische Ergebnisse. Der Luftwechselfaktor richtet sich nach der verwendeten Anlage und muss mindestens eine rückstandslose Verbrennung der Organik und die einwandfreie Nachverbrennung der Abgase gewährleisten. Bewährt hat sich hierbei ein ca. 10 facher Luftwechsel bezogen auf das verwendete Versuchsgerät. Je nach Ofentyp und Grösse kann er individuell eingestellt werden. Die Verweildauer der Teile in der Hochgeschwindigkeitsbrennkammer richtet sich nach der Grösse der Teile und der Anzahl der Luftwechsel in Verbindung mit der Dimensionierung der Ofenanlage und kann wenige Sekunden bis hin zu mehrerer Minuten betragen. In der Versuchsanlage mit einem Rohknochenmass von 2,7 cm und einer Temperatur von 650 Grad Celsius mit 10 fachem Luftwechsel waren ca. 10 Minuten als optimal anzusehen. Die Abgasnachverbrennung kann katalytisch oder thermisch erfolgen, wobei die Abwärmerückgewinnung nach dem Stand der Technik ausgeführt sein kann. Für den ersten Waschvorgang sollte Wasser reiner Qualität verwendet werden, d.h. Wasser ohne Mineralanteile und organische Verunreinigungen, wobei die Verwendung demineralisierten Wassers bereits als ausreichend zu sehen ist. Die Dauer der ersten Spülung, wenn sie durchgeführt wird, kann wenige Sekunden bis hin zu mehreren Tagen andauern. In unserem Ausführungsbeispiel hat sich eine Besprühdauer von 5 Stunden für das angestrebte Ergebnis bewährt. Das Ziel eines Kristallisationswachstumes um den Faktor 100 erreichte man mit einer Sintertemperatur von 1250 Grad Celsius und einer Haltezeit von 3 Stunden. Die Variationsmöglichkeiten liegen bei der Sintertemperatur ab 1100 Grad Celsius bis hin zur Phasenumwandlungstemperatur und bei der Haltezeit von keiner Haltezeit bis hin zu mehreren Tagen, womit die keramische Bindungsform und die Kristallitgrösse eingestellt wird. Der nachfolgende zweite Spülvorgang mit Wasser kann wenige Sekunden bis zu mehreren Tagen andauern. Für das erstrebte Ziel hat sich eine Benebelungszeit von 6 Stunden als optimal erwiesen. Wird die letzte Waschung mit sterilem Wasser durchgeführt, so ist nach dem Trockenvorgang die anschliessende Temperaturbehandlung nicht mehr nötig. Wird jedoch Wert auf Pyrogenfreiheit gelegt, so ist mit einer Minimaltemperatur von 300 Grad Celsius oder maximal mit der beginnenden Kristallitänderungstemperatur das Implantatmaterial thermisch nachzubehandeln.

Statt der Waschbehandlung kann hier auch mit dotierten Edelmetallösungen eine Integration von Metallen in die keramische Matrix erreicht werden, was zumindest im Falle von Silber zu einem wirksamen Schutz gegen Infektionen führen kann. Ansonsten ist die Beladung des fertigen keramischen Implantates mit Wirkstoffen jedweder Art nach den üblichen, dem Stand der Technik entsprechenden Methoden, möglich. So kann ein Kombinationspräparat z.B. aus Hydroxylapatitkeramik der beschriebenen Art mit Antibiotika versehen als Endprodukt resultieren, oder die Beladung mit wachstumsfördernden Präparaten, oder die sogenannte Markinokulation, oder lediglich die Tränkung mit Blut. Alle Methoden können dabei auch als Kombinationen auftreten.

Das erfindungsgemässe Verfahren kann zur Herstellung von anderen Implantatkeramikmaterial als Hydroxylapatit aufweisenden Implantatmaterial verwendet werden. Es ist auch möglich bei der Herstellung des Implantatkeramikmaterials gemäss der Erfindung die spongiösen Bereiche der Gelenke anderer Tiere als ausgewachsenen Rindern zu verwenden.

Das angegebene Verfahren kann auch für alle anderen Tierspezies verwendet werden, wobei die Zusammensetzung der resultierenden Keramik dann entsprechende Abweichungen von der Idealzusammensetzung des Hydroxylapatites aufweisen kann.

Im Besonderen kann ein derart hergestelltes Implantatmaterial auch für die in vitro Züchtung von Knorpel oder Knochengewebe, bzw. als Grundsubstanz für diagnostische Verfahren verwendet werden.

## Patentansprüche

1. Verfahren zur Herstellung von Implantatkeramikmaterial, insbesonders von Hydroxylapatit aufweisenden Implantatkeramikmaterial, **dadurch gekennzeichnet, dass** zur Herstellung die spongiösen Bereiche der Gelenke von Tieren, vorzugsweise ausgewachsenen Rindern, verwendet wird, dass die organischen Bestandteile der spongiösen Bereiche der Gelenke in einer schlagartigen Verbrennung beseitigt werden, dass eine oder mehrere Spülungen ohne chemische Zusätze von verbleibenden anorganischen Teilen des Gelenkes durchgeführt werden und dass diese anorganische Teile des Gelenkes gesintert werden.

2. Verfahren nach Anspruch 1, **dadurch gekennzeichnet, dass** zur Herstellung die metaphysären oder die epiphysären spongiösen Bereiche des Hüftgelenkes, des Kniegelenkes, der Schultergelenkes oder des Ellenbogengelenkes von Tieren, vorzugsweise Rindern, verwendet werden.

3. Verfahren nach Anspruch 1 oder 2, **dadurch gekennzeichnet, dass** Teile der Gelenke im kaltem Zustand, vorzugsweise tiefgefroren, schlagartig über ihre Zündtemperatur hinaus erhitzt werden.

4. Verfahren nach einem der vorhergehenden Ansprüchen, **dadurch gekennzeichnet, dass** die Verbrennung unter Sauerstoffüberschuss erfolgt und die Maximaltemperatur die Phasenumwandlungstemperatur des Implantatkeramikmaterials nicht überschreitet.

5. Verfahren nach einem der vorhergehenden Ansprüchen, **dadurch gekennzeichnet, dass** die schlagartige Verbrennung von wenigen Sekunden bis maximal 30 Minuten andauert.

6. Verfahren nach einem der vorhergehenden Ansprüchen, **dadurch gekennzeichnet, dass** der grösste Teil des Energiebedarfes aus der Verbrennungswärme der Organik gewonnen wird.

7. Verfahren nach einem der vorhergehenden Ansprüchen, **dadurch gekennzeichnet, dass** die entstehenden Abgase einer Abgasnachverbrennung zugeführt werden, wobei die Nachverbrennung katalytisch oder/und thermisch erfolgt und die entstehende Abwärme ebenfalls zur Prozessheizung herangezogen wird.

8. Verfahren nach einem der vorhergehenden Ansprüchen, **dadurch gekennzeichnet, dass** optional eine Spülung mit Wasser erfolgt mit der das Implantatkeramikmaterial gereinigt wird und leicht wasserlösliche Komponenten des Implantatkeramikmaterials reduziert oder/und homogenisiert werden.

9. Verfahren nach einem der vorhergehenden Ansprüchen, **dadurch gekennzeichnet, dass** als letzte Spühlung optional eine Tränkung mit Metallsatzlösungen zum bakteriellen Schutz erfolgt.

10. Verfahren nach einem der vorhergehenden Ansprüchen, **dadurch gekennzeichnet, dass** eine klassische keramische Sinterung erfolgt, und wobei die Metalle in Metallsalzlösungen keramisch gebunden werden.

11. Verfahren nach einem der vorhergehenden Ansprüchen, **dadurch gekennzeichnet, dass** optional eine Spülung mit Wasser erfolgt mit der ein Basizitätswert eingestellt wird.

12. Verfahren nach Anspruch 8 oder 11, **dadurch gekennzeichnet, dass** mindestens eine Spülung durchgeführt werden muss.

13. Verfahren nach einem der vorhergehenden Ansprüchen, **dadurch gekennzeichnet, dass** die Sinterung der anorganischen Teile des Gelenkes nach klassischen keramischen Regeln erfolgt.

14. Verfahren nach einem der vorhergehenden Ansprüchen, **dadurch gekennzeichnet, dass** das Keramikimplantat vor der Verpackung durch eine Temperaturbehandlung entpyrogenisiert wird.

15. Verfahren nach einem der vorhergehenden Ansprüchen, **dadurch gekennzeichnet, dass** das Keramikimplantat mit einem Wirkstoff oder mit Wirkstoffkombinationen versehen wird.

16. Verwendung des Implantatmateriales nach einem der vorhergehenden Ansprüchen in Verbindung mit dem Verfahren der Markinokulation.

17. Verwendung des Implantatmateriales nach einem der vorhergehenden Ansprüchen in Zellkulturen.

## Claims

1. Method for producing ceramic implant materials, preferably ceramic implant materials comprising hydroxyl apatite, **characterized by** using, for the production, the spongy parts of joints from animals, preferably grown-up cattle, removing the organic constituents of the spongy parts of the joints by a quick burning, effecting one or more flushings of the remaining inorganic parts of the joints without chemical additives and sintering of said inorganic parts of the joints.

2. Method according to claim 1, **characterized by** using, for the production, the metaphysary or epiphysary spongy parts of the hip joint, knee joint, shoulder joint or elbow joint of animals, preferably cattle.

3. Method according to claim 1 or 2, **characterized by** quickly heating parts of the joints in cold condition, preferably frozen, to above the ignition or inflammation temperature of said parts.

4. Method according to any preceding claim, **characterized by** effecting the burning at an excess of oxygen and without the maximum temperature exceeding the phase transformation temperature of the ceramic implant material.

5. Method according to any preceding claim, **characterized in that** the quick burning lasts from a few seconds to a maximum of thirty minutes.

6. Method according to any preceding claim, **characterized in that** the major part of the energy demand is obtained from the burning or combustion heat from the organic constituents.

7. Method according to any preceding claim, **characterized by** effecting an after-burning of the generated exhaust gases, whereby the after-burning is carried out catalytically and/or thermally and generated waste heat is also used for process heating.

8. Method according to any preceding claim, **characterized by** optionally carrying out flushing with water, whereby the ceramic implant material is cleaned and easily dissolvable components of the material are reduced and/or homogenized.

9. Method according to any preceding claim, **characterized by** optionally effecting as the last flushing an impregnation with metallic salt solutions for protection against bacteria.

10. Method according to any preceding claim, **characterized by** carrying through a classic ceramic sintering, whereby the metals are bonded ceramically in metallic salt solutions.

11. Method according to any preceding claim, **characterized by** optionally carrying out a flushing with water, whereby a basicity value is set.

12. Method according to any of claims 8-11, **characterized in that** at least one flushing must be carried out.

13. Method according to any preceding claim, **characterized by** effecting sintering of the inorganic parts of the joints in accordance with classic ceramic rules.

14. Method according to any preceding claim, **characterized by** depyrogenizing the ceramic implant by a temperature treatment before compression thereof.

15. Method according to any preceding claim, **characterized by** providing the ceramic implant with an active substance or with combinations of active substances.

16. Use of an implant material according to any preceding claim in connection with a mark inoculation method.

17. Use of an implant material according to any preceding claim in cell cultures.

## Revendications

1. Procédé pour la préparation d'une matière céramique destinée à un implant, en particulier une matière céramique destinée à un implant présentant de l'hydroxyapatite, **caractérisé en ce qu'**on utilise, pour la préparation, les zones spongieuses des articulations d'animaux, de préférence de boeufs arrivés à l'âge adulte, **en ce qu'**on élimine les constituants organiques des zones spongieuses des articulations en les soumettant à une brusque combustion, **en ce qu'**on procède à un ou plusieurs lavages en l'absence d'additifs chimiques des parties inorganiques de l'articulation qui subsistent et **en ce qu'**on soumet les parties inorganiques de l'articulation à un frittage.

2. Procédé selon la revendication 1, **caractérisé en ce qu'**on utilise, pour la préparation, les zones spongieuses métaphysaires ou les zones spongieuses épiphysaires de l'articulation de la hanche, de l'articulation du genou, de l'articulation de l'épaule ou de l'articulation du coude d'animaux, de préférence de boeufs.

3. Procédé selon la revendication 1 ou 2, **caractérisé en ce qu'**on soumet des parties des articulations à l'état froid, de préférence à l'état congelé, à un chauffage brutal au-delà de leur température d'inflammation.

4. Procédé selon l'une quelconque des revendications précédentes, **caractérisé en ce que** la combustion a lieu en présence d'un excès d'oxygène et **en ce que** la température maximale ne dépasse pas la température de transformation de phase de la matière céramique destinée à un implant.

5. Procédé selon l'une quelconque des revendications précédentes, **caractérisé en ce que** la durée de la combustion brusque s'étend de quelques secondes à un laps de temps maximal de 30 minutes.

6. Procédé selon l'une quelconque des revendications précédentes, **caractérisé en ce que** la majeure partie de la demande en énergie est récupérée de la chaleur de combustion des composés organiques.

7. Procédé selon l'une quelconque des revendications précédentes, **caractérisé en ce que** les gaz d'échappement que l'on obtient sont acheminés à une combustion ultérieure des gaz d'échappement, la combustion ultérieure ayant lieu par voie catalytique et/ou par voie thermique et les chaleurs perdues obtenues étant également utilisées pour le chauffage du processus.

8. Procédé selon l'une quelconque des revendications précédentes, **caractérisé en ce que**, le cas échéant, on procède à un rinçage avec de l'eau, avec lequel on nettoie la matière céramique destinée à un implant et on soumet à une réduction et/ou à une homogénéisation les composants aisément hydrosolubles de la matière céramique destinée à un implant.

9. Procédé selon l'une quelconque des revendications précédentes, **caractérisé en ce que**, à titre de dernier rinçage, on procède le cas échéant à une imprégnation avec des solutions de sels métalliques à des fins de protection contre les bactéries.

10. Procédé selon l'une quelconque des revendications précédentes, **caractérisé en ce qu'**on procède à un frittage céramique classique, les métaux étant soumis à une liaison céramique dans les solutions de sels métalliques.

11. Procédé selon l'une quelconque des revendications précédentes, **caractérisé en ce qu'**on procède le cas échéant à un rinçage avec de l'eau, avec lequel on règle une valeur de basicité.

12. Procédé selon la revendication 8 ou 11, **caractérisé en ce que** l'on doit procéder à au moins un rinçage.

13. Procédé selon l'une quelconque des revendications précédentes, **caractérisé en ce que** le frittage des parties inorganiques de l'articulation a lieu conformément à des règles céramiques classiques.

14. Procédé selon l'une quelconque des revendications précédentes, **caractérisé en ce qu'**on soumet l'implant en céramique, avant son conditionnement, à une dépyrogénéisation via un traitement thermique.

15. Procédé selon l'une quelconque des revendications précédentes, **caractérisé en ce que** l'implant en céramique est muni d'une substance active ou d'une combinaison de substances actives.

16. Utilisation de la matière d'implant selon l'une quelconque des revendications précédentes en liaison avec le procédé de l'inoculation de moelle.

17. Utilisation de la matière d'implant selon l'une quelconque des revendications précédentes dans des cultures cellulaires.
